# EUROPEAN PATENT SPECIFICATION

(11) **EP 4 321 139 B1**
(45) Date of publication and mention of the grant of the patent: **06.05.2026**
(21) Application number: 23161564.2
(22) Date of filing: 13.03.2023
(51) Int. Cl.: A61F 13/494, A61F 13/496

(54) **ABSORBENT ARTICLE WITH TRANSVERSAL BARRIER**
ABSORBIERENDER ARTIKEL MIT QUERBARRIERE
ARTICLE ABSORBANT À BARRIÈRE TRANSVERSALE

(30) Priority: 09.08.2022 EP 22189516
(43) Date of publication of application: 14.02.2024
(73) Proprietor: Ontex BV, 9255 Buggenhout (BE); Ontex Group NV, 9320 Erembodegem (BE)
(72) Inventor: HEEGE, Thomas, 56761 Düngenheim (DE); IFFLAND, Dirk, D-45529 Hattingen (DE); BUYSSE, Michiel, 9968 Oosteeklo (BE); PSCHYKLENK, Lukas, 56727 Müllenbach (DE); FRANSEN, Markus, 56269 Dierdorf (DE)
(74) Representative: Macchetta, Andrea

(56) References cited:
- WO-A1-2014/002440
- US-A1- 2012 022 492
- US-A1- 2018 338 870
- US-A1- 2021 205 148
- US-B1- 6 221 460
- US-B2- 10 159 611
- US-B2- 7 204 830

## Description

### TECHNICAL FIELD

The present disclosure is directed to an absorbent article for personal hygiene, typically in the form of pants (such as for babies, youths or adults) and generally of the disposable kind.

### BACKGROUND

Absorbent articles for personal hygiene are designed to absorb and contain bodily exudates, such as a large quantity of urine and/or stool. Non-limiting examples of disposable absorbent articles include diapers, pants, training pants, pADL, adult incontinence products, and feminine hygiene products (including, for example, sanitary napkins and tampons). Other examples of disposable absorbent articles include bandages and wound dressings. In some embodiments, for example, an absorbent article comprises several layers providing different functions, for example a topsheet, a backsheet and in-between an absorbent core, among other layers.

The function of the absorbent core is to absorb and retain the exudates for a prolonged amount of time, for example overnight for a diaper, minimize re-wet to keep the wearer dry and avoid soiling of clothes or bed sheets. The majority of currently marketed absorbent articles comprise as absorbent material a blend of comminuted wood pulp (also referred to as fluff pulp and/or cellulose fibers) with superabsorbent polymers (SAP) in particulate form, also called absorbent gelling materials (AGM), see for example U.S. Pat. No. 5,151,092 (Buell). Absorbent articles having a core consisting essentially of SAP as absorbent material (so called "airfelt-free" cores) have also been proposed but are less common than traditional mixed cores (see e.g. WO2008/155699 and WO2012/052172).

A number of disclosures exist (see for example EP3451989 B1) directed to limiting the risk of leakage by providing transversal barriers at the front and/or back of a diaper. However whilst such executions may be effective on diaper constructs they are less effective and or complex/costly to incorporate into a pant concept.

An attempt to overcome such shortcomings has been made in for example WO2021/170027 A1 wherein a nonwoven layer of the front and/or back belt is folded a plurality of times to form a waist guard. Although this provides for an effective way to introduce a transversal barrier in a pant construct, there are several drawbacks that have been identified: firstly, the nonwoven that is generally hydrophobic may be sufficient to capture solids but is less effective to capture larger amounts of liquid exudates (particularly under pressure such as when a baby is moving and/or application of sudden pressure such as sitting/dropping on the floor on its buttocks); secondly it limits the positioning thereof to a closer position at the waist edge which is less effective in providing a barrier since it is positioned further away from the core and to position it closer to the core and away from the waist edge would require a significant increase in length of the nonwoven which adds significant waste and cost; thirdly applying the multiple folds at different positions of the same component and especially further elasticizing the region when/if desired may add significant process complexity particularly at high speeds.

A need therefore exists for improved absorbent articles having leakage protection particularly for substantially liquid exudates such as urine and/or liquid stool yet in in a cost effective manner and with more limited additional waste.

### SUMMARY

The invention is as defined in the appended claims.

In a first aspect the disclosure relates to an absorbent article for personal hygiene, preferably a disposable pant, comprising: a front panel preferably substantially transversely extending; a back panel preferably substantially transversely extending; and an absorbent insert, preferably substantially longitudinally extending, joined to each of said front and back panels, said absorbent insert comprising front and back transversal edges and left and right longitudinal edges connecting the front and back transversal edges to form a perimeter thereof and generally wherein the left and right longitudinal edges extend substantially along the longitudinal axis y and are oppositely disposed such that said longitudinal axis y extends therebetween, and the front and back transversal edges extend substantially along the transversal axis x, and typically substantially perpendicular to the longitudinal axis y, and are oppositely disposed such that said transverse axis x extends therebetween). The front and back panels are joined together to define a pair of side seams, and at least a portion of said absorbent insert proximal to the front and/or back transversal edges thereof comprises a transversal waist barrier comprising a substantially liquid impermeable film layer, preferably wherein said transversal waist barrier (5) is an up-standing barrier generally arranged to block substantially liquid exudates from flowing therethrough.

The film may be elastic (or may comprise elastic material such as one or more elastic strands and/or elastic foam) and may comprise micro-openings sized to allow at least some water vapour to permeate therethrough. Preferably the film having a ratio of the MD load at break to the CD load at break of less than about 8, and a machine-direction notched trapezoidal tear strength of at least about 25 g.

In a second aspect the disclosure relates to a method for the manufacture of an absorbent article comprising the steps of: providing a front panel; providing a back panel; providing an absorbent insert comprising front and back transversal edges and left and right longitudinal edges connecting the front and back transversal edges to form a perimeter thereof and generally wherein the left and right longitudinal edges extend substantially along the longitudinal axis y and are oppositely disposed such that said longitudinal axis y extends therebetween, and the front and back transversal edges extend substantially along the transversal axis x, and typically substantially perpendicular to the longitudinal axis y, and are oppositely disposed such that said transverse axis x extends therebetween); folding a portion of said absorbent insert proximal to the front and/or back transversal edges thereof onto itself, in the shape of a substantially U-fold, to form transversal waist barrier(s); joining said insert to said front and back panels; and joining said front and back panels together along a pair of oppositely disposed side seams.

### BRIEF DESCRIPTION OF THE FIGURES

**FIG. 1** is a top planar view of an absorbent article according to an embodiment of the present disclosure.
**FIG. 2** is a top planar view of an absorbent article according to an embodiment of the present disclosure.
**FIG. 3** is a partial cross-section of an absorbent article according to an embodiment of the present disclosure.
**FIG. 4** is a top planar view of an absorbent article according to an embodiment of the present disclosure.

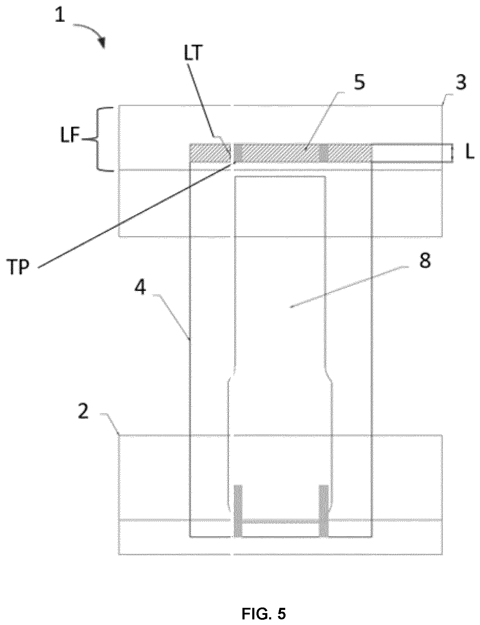

**FIG. 5** is a top planar view of an absorbent article according to an embodiment of the present disclosure.

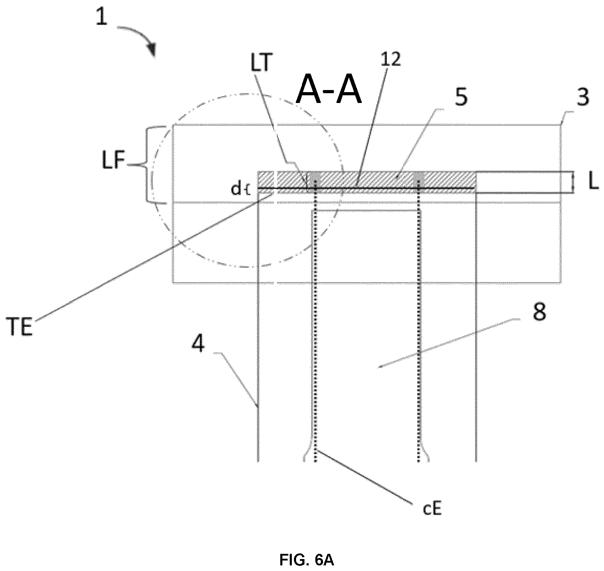

**FIG. 6A** is a partial top planar view of an absorbent article according to an embodiment of the present disclosure.

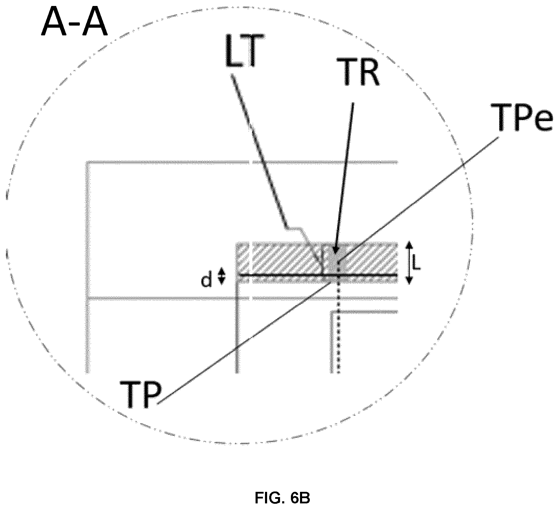

**FIG. 6B** is an enlarged view of the indicated portion A-A of Fig. 6A.
**FIG. 7** is a perspective view of an absorbent article according to an embodiment of the present disclosure.
**FIG. 8** is a perspective view partial-section of an absorbent article according to an embodiment of the present disclosure.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" refers is itself also specifically disclosed.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints unless otherwise stated.

As used herein, the "skin facing", "body-facing" or "bodyside" surface means that surface of the article or component which is intended to be disposed toward or placed adjacent to the body of the wearer during ordinary use, while the "outward", "outward-facing" or "garment-side" or "garment facing" surface is on the opposite side, and is intended to be disposed to face away from the wearer's body during ordinary use. Such outward surface may be arranged to face toward or placed adjacent to the wearer's garments or undergarments when the absorbent article is worn.

As used herein, the term "absorbent article" refers to disposable devices such as infant or adult diapers or pads, pants, training pants, and the like which are placed against or in proximity to the body of the wearer to absorb and contain the various exudates discharged from the body. Typically these articles comprise a topsheet, backsheet, an absorbent core and optionally an acquisition system (which may be comprised of one or several layers) and typically other components, with the absorbent core normally placed between the backsheet and the acquisition system or topsheet.

The absorbent articles of the disclosure will be further illustrated in the below description and in the Figures, though all embodiments described herein may equally be applied onto absorbent articles in the form of pants (or even in the form of feminine hygiene articles such as menstrual pants and/or slips/panties). Nothing in this description should be however considered limiting the scope of the claims unless explicitly indicated otherwise. Unless indicated otherwise, the description refers to the dry article, i.e. before use and conditioned at least 24 hours at 21° C.+/-2° C. and 50+/-20% Relative Humidity (RH).

A "nonwoven web" as used herein means a manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yarn). Nonwoven webs can be formed by many processes such as meltblowing, spunbonding, solvent spinning, electrospinning, carding and airlaying. The basis weight of nonwoven webs is usually expressed in grams per square meter (g/m2 or gsm).

The terms "joined" or "bonded" or "attached", as used herein, encompasses configurations whereby an element is directly secured to another element by affixing the element directly to the other element, and configurations whereby an element is indirectly secured to another element by affixing the element to intermediate member(s) which in turn are affixed to the other element. The terms further include embodiments in which a pocket or other connector is formed in or attached to an area of the absorbent article. Further, these terms include configurations in which the elements are removably, or non-removably, joined, bonded, or attached. For example, wherein an element is described as "joined" within the configuration, it may be either removably joined or non-removably joined unless otherwise specified or evident from the context.

The terms "comprise," "comprising," and "comprises" are open ended terms, each specifies the presence of what follows, e.g., a component, but does not preclude the presence of other features, e.g., elements, steps, components known in the art, or disclosed herein. These terms based on the verb "comprise" should be read as encompassing the narrower terms "consisting of" which excludes any element, step, or ingredient not specified and "consisting essentially of" which limits the scope of an element to the specified materials or steps and those that do not materially affect the way the element performs its function. Any preferred or exemplary embodiments described below are not limiting the scope of the claims, unless specifically indicated to do so. The words "typically", "normally", "advantageously" and the likes also qualify elements which are not intended to limit the scope of the claims unless specifically indicated to do so.

By "absorbent material" it is meant a material which has some absorbency property or liquid retaining properties, such as SAP, cellulosic fibers as well as synthetic fibers, most preferably is selected from the group consisting of SAP, cellulose (or cellulosic) fibers, and mixtures thereof. Herein, absorbent materials in the form of fibrous absorbent materials have been found to be useful. These fibrous absorbent materials can comprise or consist of natural fibers, e.g. cellulosic fibers as well as synthetic fibers. Typically, glues used in making absorbent cores have no absorbency properties and are not considered as absorbent material.

"Tear strength" or "tear force," reflects the ease or difficulty by which the film can be torn, and is expressed in units of grams. Herein, tear strength may be measured by the Elmendorf notched tear test, ASTM D-1922, incorporated herein by reference and/or by the Trapezoid tear test ("trap test"), as described herein or according to ASTM D-5587. The test may be performed with either a notched or an unnotched film and in either the CD or MD direction. Unless otherwise specified, herein tear strength is notched tear strength. It is noted that tear strength is related to film thickness, and any comparison of tear strengths must take into account the relative basis weights of the comparative samples.

"Tensile strength," as used herein means the load required to induce a break ("load at break") in the film in either the CD or the MD. Tensile strength is expressed in units of N/cm or equivalent units thereof, and is determined by ASTM method D822-02, using the following parameters: Sample Direction = MD x CD; Sample size = 1 inch width x 6 inch length; Test speed = 20 in/min; Grip distance = 2 inch. Grip size = 3 inch wide rubber faced grips evenly gripping sample.

As used herein, the term "absorbent core" refers to the component or components of the article having the most absorbent capacity and comprising an absorbent material and optionally a core wrap enclosing the absorbent material. The term "absorbent core" does not include the acquisition-distribution system or layer or any other component of the article which is not either integral part of the core wrap or placed within the core wrap. The core may consist essentially of, or consist of, a core wrap, absorbent material as defined below and glue enclosed within the core wrap.

The values indicated herein are measured according to the methods indicated herein below, unless specified otherwise. All measurements are performed at 21±2° C. and 50±20% RH, unless specified otherwise. All samples should be kept at least 24 hours in these conditions to equilibrate before conducting the tests, unless indicated otherwise. All measurements should be reproduced on at least 4 samples and the average value obtained indicated, unless otherwise indicated.

### THE ABSORBENT ARTICLE

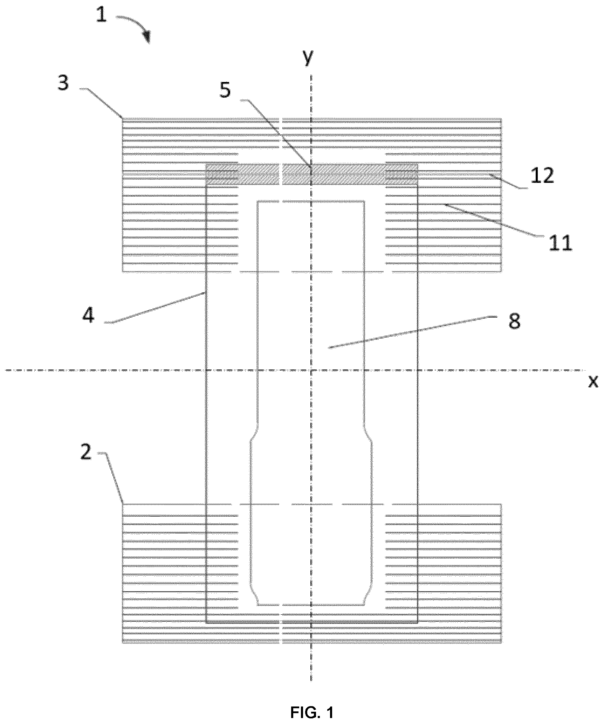

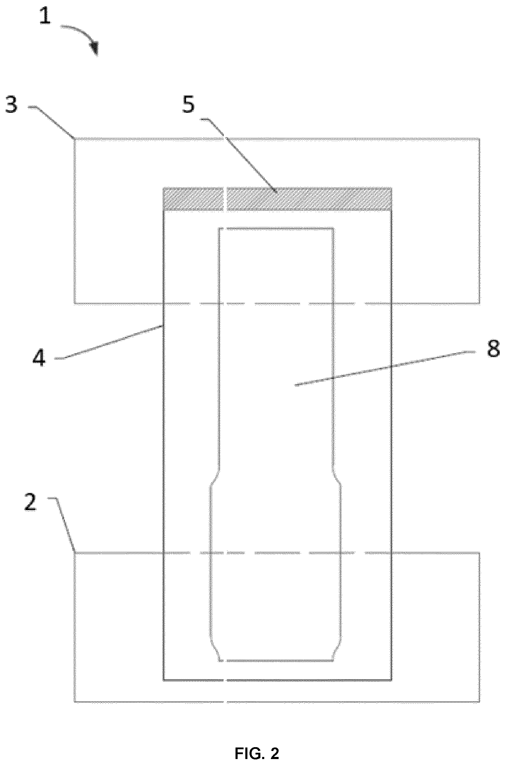

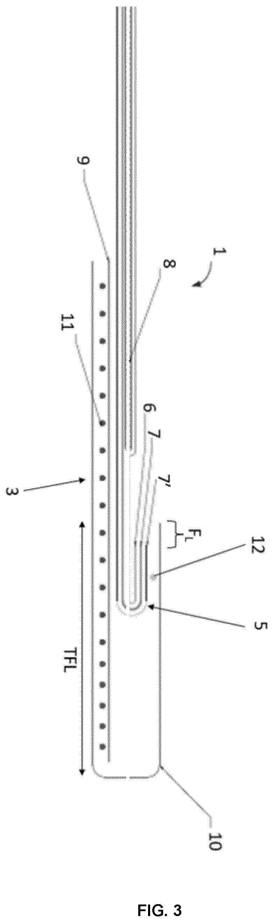

As exemplified in Fig.1 to Fig.3, absorbent articles herein are for personal hygiene, preferably in the form of a pant, the absorbent article comprising: a substantially transversely extending front panel (2); a substantially transversely extending back panel (3); and a substantially longitudinally extending absorbent insert (4) joined to each of said front and back panels (2, 3), said absorbent insert (4) comprising front and back transversal edges and left and right longitudinal edges connecting the front and back transversal edges to form a perimeter thereof (generally wherein the left and right longitudinal edges extend substantially parallel to the longitudinal axis y and are oppositely disposed such that said longitudinal axis y extends therebetween, and the front and back transversal edges extend substantially parallel to the transversal axis x, and typically substantially perpendicular to the longitudinal axis y, and are oppositely disposed such that said transverse axis x extends therebetween); wherein the front and back panels (2, 3) are joined together to define a pair of side seams, and wherein at least a portion of said absorbent insert (4) proximal to the front and/or back transversal edges thereof comprises a transversal waist barrier(s) (5) comprising a substantially liquid impermeable film layer, preferably wherein said transversal waist barrier (5) is an up-standing barrier generally arranged to block substantially liquid exudates from flowing therethrough, and generally wherein said insert (4) comprises one or more folds forming the transversal waist barrier (5) (wherein by "forming" the transversal waist barrier it is meant that at least a portion of the barrier is achieved by folding the insert typically as described herein, said portion preferably being that coming into most contact with exudates). Advantageously, this arrangement allows for an absorbent article with superior leakage prevention particularly for substantially liquid exudates such as urine and/or liquid stool.

The liquid impermeable film layer is breathable and has a basis weight of less than 18 g/m² and wherein the impermeable film layer forms an integral part of the insert, preferably has a basis weight of less than 16 g/m², preferably from 5 g/m² to 15 g/m², more preferably from 8 g/m² to 14 g/m². The film may be elastic (or may comprise elastic material such as one or more elastic strands and/or elastic foam) and may comprise micro-openings sized to allow at least some water vapour to permeate therethrough. Preferably the film having a ratio of the MD load at break to the CD load at break of less than about 8, and a machine-direction notched trapezoidal tear strength of at least about 25 g. Advantageously this allows for useful flexibility and comfort to the skin.

When the liquid impermeable film layer is a discrete layer and generally applied in the form of a patch joined to said insert via suitable bonding techniques (such as adhesive and/or mechanical bonding as generally described in more detail herein), said film is preferably elastic. "Elastic," "elastomeric," and "elasticized/elasticised" herein generally mean the ability of a material to stretch by at least 100% without rupture or breakage at a given load, and upon release of the load the elastic material or component exhibits at least 70% recovery (i.e., has less than 30% set) in one of the directions as per the Hysteresis Test described herein. Stretch, sometimes referred to as strain, percent strain, engineering strain, draw ratio, or elongation, along with recovery and set may each be determined according to the Hysteresis Test described in more detail below. Materials that are not elastic are referred as inelastic. "Extensible" typically means the ability to stretch or elongate, without rupture or breakage, by at least 50% as per step 5(a) in the Hysteresis Test herein (replacing the specified 100% strain with 50% strain). In this embodiment, it may be preferable that the liquid impermeable film layer is joined to the insert by a combination of adhesive and mechanical bonding.

The adhesive may be applied in a first joining zone and the mechanical bonding may be applied on a second joining zone, and preferably wherein the adhesive is continuously applied along the first joining zone at an area generally comprising substantially the entire transversal-waist-barrier-width (typically extending along an axis parallel to the transversal axis x, and generally adjacent the front and/or back transversal edges). The first and second joining zones may be proximal to each other (or adjacent) such that the majority, preferably substantially all, the area of adhesive is not overlapped by mechanical bonds. Advantageously this allows for extra anchoring and breathability generally afforded by the mechanical bonding yet maintain an appropriate liquid seal to limit leakage through the barrier along the transversal edge(s) of the insert.

The impermeable film layer may be discrete and joined to the insert (4) by at least one of adhesive and mechanical bonding (such as ultrasonic bonding, heat bonding, pressure bonding, and the like); or may be an integral part of the insert (4) and may form barriers herein such as by folding a portion thereof as described in more detail hereinbelow.

Generally, the substantially liquid impermeable film layer is an integral component of at least one element or portion of the absorbent article such as a backsheet (7) thereof typically such that no additional and/or separate layer, element and/or material is incorporated to form the transversal waist barrier(s) (5). Advantageously this allows to reduce waste and/or cost associated with incorporating separate elements to create the transversal waist barrier(s).

The backsheet (7) is generally that portion of the absorbent article positioned adjacent the garment-facing surface of the absorbent core (8) and which prevents the exudates absorbed and contained therein from soiling articles such as bedsheets and undergarments. The backsheet (7) is typically impermeable to liquids (e.g. urine). The backsheet (7) may for example be or comprise a thin plastic film such as a thermoplastic film having a thickness of about 0.012 mm to about 0.051 mm. Preferably the basis weight of the backsheet (7) is less than or equal to 16 g/m², more preferably from 10 g/m² to 14 g/m². Exemplary backsheet films include those manufactured by Tredegar Corporation, based in Richmond, Va., and sold under the trade name CPC2 film. Other suitable backsheet materials may include breathable materials which permit vapors to escape from the absorbent article while still preventing exudates from passing through the backsheet (7). Exemplary breathable materials may include materials such as woven webs, nonwoven webs, composite materials such as film-coated nonwoven webs, microporous films such as manufactured by Mitsui Toatsu Co., of Japan under the designation ESPOIR NO and by Tredegar Corporation of Richmond, Va., and sold under the designation EXAIRE, and monolithic films such as manufactured by Clopay Corporation, Cincinnati, Ohio under the name HYTREL blend P18-3097. Some breathable composite materials are described in greater detail in PCT Application No. WO 95/16746 published on Jun. 22, 1995 in the name of E. I. DuPont; U.S. Pat. No. 5,938,648 to LaVon et al., U.S. Pat. No. 4,681,793 to Linman et al., U.S. Pat. No. 5,865,823 to Curro; and U.S. Pat. No. 5,571,096 to Dobrin et al, U.S. Pat. No. 6,946,585B2 to London Brown.

In a preferred embodiment the backsheet (7) is breathable. Breathable backsheet herein typically means that it comprises micro-openings sized to allow at least some water vapour to permeate through the backsheet that typically comprises a film such as a polyethylene (PE) film.

Preferably, the backsheet (7) is breathable and has a basis weight in the ranges described herein above, and/or preferably of less than 15 g/m², preferably from 8 g/m² to 14 g/m². The backsheet preferably comprises (or consists of) a thermoplastic film having a ratio of the MD load at break to the CD load at break of less than about 8, and a machine-direction notched trapezoidal tear strength of at least about 25 g. Preferably, wherein the MD load at break is at least about 2.0 N/cm and the CD load at break is at least about 0.7 N/cm. Advantageously low basis weight films are particularly useful for folding and forming exudate barriers herein because of their added flexibility and comfort to the skin, yet sufficient mechanical strength is desirable for processability on converting lines as well as resisting folding strains especially in embodiments herein where an elastic is further applied onto the barrier to form an active stand-up gather. Suitable films are for example commercially available materials sold by RKW SE (Havellandstraße 8, 68309 Mannheim, Germany) under the name HyCare M-15.

The film may further have an opacity of at least about 50%. Preferably wherein said film is substantially void of titanium dioxide; and/or wherein the film comprises an olefin block copolymer which is ethylene-based or propylene-based or combinations thereof; and/or wherein said film comprises from about 30% to about 60% by weight of a filler; and/or wherein the film is a coextruded multilayer film; and/or wherein the film is a monolayer film. Advantageously this allows for identification of the barrier without the use of significant amounts of pigment.

Preferably a backsheet (7) is breathable when the water vapour transmission rate (WVTR) of the backsheet is at least 500 grams/m2 - 24 hours, preferably at least 1,000 grams/m2 - 24 hours, even more preferably from 1,200 grams/m2 - 24 hours to 2,500 grams/m2 - 24 hours, even more preferably from 1,500 grams/m2 - 24 hours to 2,100 grams/m2 - 24 hours, as measured according to ASTM D6701-21.

The backsheet (7) may be joined to the topsheet (6), the absorbent core (8) or any other element of the absorbent article by any attachment means known in the art. Suitable attachment means are described above with respect to means for joining the topsheet to other elements of the article. For example, the attachment means may include a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minn. and marketed as HL-1620 and HL 1358-XZP. Alternatively, the attachment means may comprise heat bonds, pressure bonds, ultrasonic bonds, dynamic mechanical bonds, or any other suitable attachment means or combinations of these attachment means as are known in the art.

The absorbent core (8) of the absorbent article may comprise one or more core layers. As explained, the absorbent article may comprise an acquisition distribution system, which will typically consist of one or more layers. Most typically, the layers are arranged above the core layer. Hence, a number of layers can be arranged between the topsheet and the backsheet. The skilled person will usually have no difficulty in distinguishing between these layers. In case of doubt, a core layer can be identified as being a layer which is generally less permeable than a layer forming part of the acquisition-/distribution-system.

Permeability generally refers to the quality of a porous material that causes it to a lower liquid or gases to pass through it. Hence, the layers of the acquisition distribution system should generally be more permeable than the layers of the core system as these layers are meant to distribute liquid to the absorbent core, where the liquid is ultimately stored.

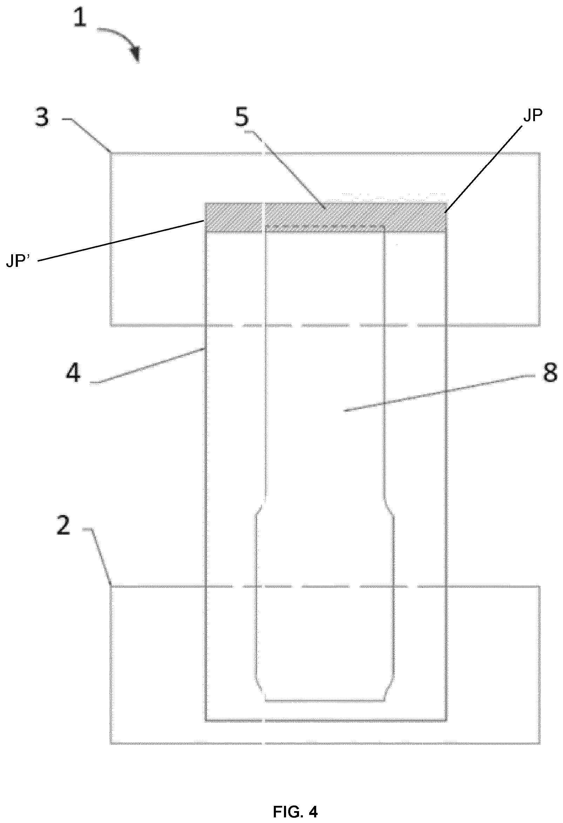

In an embodiment, as exemplified in Fig. 4, the transversal waist barrier(s) (5) overlap at least a portion of an absorbent core (8) of the insert (4) typically such that the exudates collected and/or retained by the transversal waist barrier(s) (5) are substantially dehydrated and/or absorbed by the neighbouring absorbent core (8) surface(s). Advantageously this allows for reduced leakage risks of highly liquid exudates like urine or liquid stool such as diarrhoea (or other liquid stool that is generally and commonly observed e.g. with newborn babies).

Preferably the front and back panels (2, 3) are separate and connected to each other by the insert (4) acting as a bridge element to form a substantially H-shaped pant. Advantageously this allows to omit cutting/shaping of panels in a crotch region of the article thus reducing waste and/or limiting the amount of panel material in view of its absence in the crotch region also helping to reduce waste and cost.

The insert (4) may be joined to the front and back panels (2, 3) by adhesive and/or mechanical bonding such as ultrasonic bonding, pressure bonding, and/or thermal bonding and the like. In a preferred embodiment the insert (4) is joined to the front and back panels (2, 3) by a discontinuous pattern of adhesive. Advantageously this may help to retain softness and pliability of the laminated structures and reduce stiffness when worn.

In an embodiment, the substantially liquid impermeable film layer comprises, preferably consists of, a backsheet (7) of the absorbent insert (4) and preferably comprises a material comprising, or consisting of, a polyester, more preferably said material being selected from the group consisting of polyethylene, polylactic acid, and polypropylene. Advantageously such transversal waist barrier(s) are not only impermeable to a much higher degree than hydrophobic nonwovens but further are formed by using a component already present in the absorbent article and hence does not require adding any further materials that would impact waste and/or cost.

Preferably, the at least portion of said absorbent insert (4) proximal to the front and/or back transversal edges thereof comprises one or more folds forming the transversal waist barrier(s) (5), preferably wherein said fold is substantially U-shaped. Advantageously such fold of the insert allows to form a liquid impermeable barrier to exudates, and particularly the U-shape (vs other shapes) allows to form a maximised pocket for exudate collection with the minimum use of material.

In a preferred embodiment, a portion of the insert proximal (typically adjacent) to the front and/or back transversal edges thereof may be folded onto itself (and that is sometimes referred to as an insert "in folded state") and the fold (generally as described in embodiments herein) has a length (L) in a longitudinal direction running substantially parallel to a longitudinal axis (y), and wherein said insert (4) further comprises a pair of longitudinally extending cuffs positioned along left and right longitudinal edges thereof and joined thereto along a tack-down length (LT) extending from a position proximal (preferably adjacent) to at least the back transversal edge to a tack-down terminal position (TP) typically longitudinally displaced therefrom along said longitudinal axis (y) (generally when viewed in a planar direction with the insert in an un-folded state), and wherein the tack-down length (LT) is less than or equal to about 2L, preferably said cuffs being joined to a body-facing surface of the topsheet (6). It is understood herein that whilst this embodiment makes specific reference to the "back" transversal edge, when the transversal waist barrier(s) (5) are positioned at the front of the article, the same dimensional characteristics apply with respect to the "front" transverse edge. Advantageously, this allows for a reduced tack-down length that permits to maximise the volume of the containment pocket formed by extending the lateral cavities when the fold stands up in contact with the user when the article is worn.

The tack-down region (TR) may extend along an axis that is parallel to the longitudinal axis (y) from a position proximal (preferably adjacent) to the back and/or front transversal edge to a tack-down terminal position (TP) and may comprise adhesive substantially in the form of a continuous stripe (and/or plural stripes). The terminal position (TP) may be congruent with the back and/or front transversal edge (generally with the insert in a folded state when viewed in a planar direction, as can be generally seen in the figures), this is generally the case when the tack-down length (LT) is equal to about 2L. Alternatively, the terminal position (TP) may be positioned inboard of the back and/or front transversal edge when viewed in a planar direction typically when being in the same folded state described above (generally meaning that it is positioned further away from a transverse centerline of the article and/or insert compared to the front and/or rear transversal edge along a longitudinal direction substantially parallel to the longitudinal axis y), this is generally the case when the tack-down length (LT) is less than 2L.

The back and/or front transversal edge (generally in folded state, as can be generally seen in the figures) typically coincides with a terminal edge (TE) of the transversal waist barrier (5) forming a pocket opening.

In a preferred embodiment, the tack-down length (LT) is from 0.5L to 2L, preferably from 0.6L to 1.9L, even more preferably from 0.7L to 1.5L, most preferably from 0.8L to 1.2L. Advantageously this allows for the desirable increased volume capacity of the pocket whilst retaining good cuff sealing as well as cuff lift-off at a position proximal to the fold of the insert such as to attain a 180° all-round barrier to exudates (viewed in a planar direction).

In an embodiment, the insert (4) comprises a pair of longitudinally extending cuffs positioned along left and right longitudinal edges thereof, wherein each cuff comprises at least one elastic (cE), and wherein at least a terminal portion of said elastic (TPe) overlaps with the transversal waist barrier (5). Generally the said terminal portion (TPe) comprising, or consisting of, a terminal end of the said elastic. Advantageously this allows the cuffs to stand-up at a cuff lift-off position that is inboard of the pocket such as to attain a 180° all-round barrier to exudates (generally when viewed in a planar direction).

Preferably, the transversal waist barrier (5) comprises one or more second elastic strands (12) positioned at a distance (d) from a terminal edge (TE) of the transversal waist barrier (5) forming a pocket opening, and wherein the distance between the elastic closest to said terminal edge (TE) and said terminal edge is less than about 0.6L, preferably from 0.15L to 0.5L, even more preferably from 0.20 to 0.45L. Advantageously this permits the transversal barrier to more easily stand-up and make a seal with the wearer's skin and preferably yet be sufficiently displaced from the terminal edge to minimize risk of elastic exposure to the skin of a wearer.

The one or more second elastic strands (12) of the transversal waist barrier (5) may have a first dtex and the front and back panels (2,3) may comprise a plurality of elastics (preferably in the form of a plurality of first elastic strands) having a second dtex, wherein the first dtex is greater than the second dtex, and preferably wherein the second dtex is from 300dtex to 1200dtex, preferably 350dtex to 950dtex, preferably 500dtex to 900dtex, preferably from 600dtex to 800dtex. Advantageously this permits to attain a tension differential for better seal behaviour when the article is worn and in tension.

In an embodiment, the one or more second elastic strands (12) have a dtex (i.e. a first dtex) of from 600dtex to 1500dtex, preferably from 650dtex to 1400dtex, more preferably from 700dtex to 1300dtex, even more preferably from 800dtex to 1200dtex.

In an embodiment, at least one of the second elastic strands is a flat elastic. By "flat elastic" it is meant an elastic having a substantially line-form cross-section compared to classic elastics having circular cross-section. Advantageously this allows for a softer to the touch comfort whilst attaining a higher tensile force.

Preferably, the folded portion of said absorbent insert (4) is joined to the body-facing surface of the topsheet (6) at a pair of oppositely disposed extremities of the insert (4) along a portion of the left and right longitudinal edges thereof, generally wherein said oppositely disposed extremities are connected by a fold of said folded portion of said absorbent insert (4), such that a pocket is formed that acts as a barrier to exudates in both the longitudinal direction (i.e. substantially parallel to the longitudinal axis y) and the transverse direction (i.e. substantially parallel to the transverse axis x). The said extremities of the insert (4) may be joined by adhesive and/or mechanical bonding such as ultrasonic bonding, thermal bonding, and/or pressure bonding and the like.

In an embodiment, the absorbent insert (4) comprises a substantially liquid permeable topsheet (6), a substantially liquid impermeable backsheet (7), and an absorbent core (8) sandwiched therebetween, preferably wherein the absorbent core (8) is positioned inboard of the perimeter of said absorbent insert (4) when viewed in a planar direction (as generally depicted and illustrated in the figures herein and that typically corresponds to a plane formed by the x and y axis described and illustrated herein) such that the one or more folds are substantially free of absorbent material. Advantageously, a fold that is substantially free of absorbent material allows for a more flexible fold that can allow the barrier to better stand upright upon application of tension and yet limit piercing of the topsheet and/or backsheet that could arise if exerting a folding force in an area of the insert comprising SAP particles.

In an embodiment, the font and/or back panels (2, 3) are elastic and comprise an inner nonwoven layer (9), an outer nonwoven layer (10), and an elastic material (11) sandwiched therebetween, wherein the elastic material is selected from the group consisting of an elastic film and a plurality of elastic strands. Preferably, the outer nonwoven layer (10) is wider than the inner nonwoven layer (9) (generally in a direction substantially parallel to the longitudinal axis y) and is folded over said inner nonwoven layer (9) such that it overlaps the portion of said absorbent insert (4) proximal to the front and/or back transversal edges and generally over a fold length (LF) (also sometimes referred to herein as total fold length (TFL)), preferably such that said outer nonwoven layer (10) overlaps both a portion of the backsheet (7) and a portion of the topsheet (6) that remains exposed from said one or more transversal waist barrier(s) (5) such to form a flange (F_{L}). Advantageously this allows to cover the backsheet with nonwoven so that the backsheet does not come into contact with the skin of a wearer which could otherwise result in skin irritations and/or less comfort.

The inner and outer nonwoven layers (9, 10) may be the same or different meaning that they may have the same or different properties selected from basis weight (in gsm) and composition. In an embodiment, both the inner and outer nonwoven layers (9, 10) each comprise a spunbond nonwoven, typically comprising monocomponent fibers of polypropylene, and each have a basis weight of from 10 g/m² (gsm) to 30 g/m² (gsm), preferably from 12 g/m² (gsm) to 25 g/m². The outer nonwoven layer (10) may have a basis weight that is higher than the inner nonwoven layer (9). Advantageously, although it is customary to have the higher basis weight nonwoven on the inner nonwoven wearer skin facing side, in barrier embodiments herein (especially when the outer nonwoven layer is folded and/or extends over the insert; and/or when the transversal barrier is elasticised) such arrangement provides for added mechanical integrity desirable for resistance to tear on elastification and/or added softness for the wearer on the front and/or back waist regions up to the barrier location.

Preferably, and as exemplified in Fig. 1, the elastic material (11), such as the plurality of elastic strands, is/are deactivated in an area of overlap of the insert (4) when viewed in a planar direction. The deactivation is preferably achieved by cutting of the elastic strands such as to render the deactivated region of the panel(s) (2, 3) substantially inelastic and regions neighbouring said deactivated region and comprising said elastic strands being elastic. Typically the absorbent core (8) of the insert (4) is positioned within the deactivated region such that it substantially does not overlap the elastic strands (11). Advantageously this allows for improved core integrity as risks of core collapse due to excessive tension from the stretched elastics when the article is worn is limited.

Preferably more than 55%, preferably more than 60%, even more preferably from 65% to 95%, of the total surface area (generally when viewed in a planar direction) of the transversal waist barrier(s) (5) is positioned to overlap the deactivated region of the panel(s) (2, 3). Advantageously this allows for improved fit of the barrier when the article is worn by a subject.

In addition or alternatively, more than 50%, preferably more than 60%, even more preferably from 65% to 95%, of a total transversal length (generally substantially parallel to the transversal axis x) of the transversal waist barrier(s) (5) overlaps the deactivated region of the panel(s) (2, 3). Especially when the transversal waist barrier(s) is elastic, this arrangement may advantageously not only improve core integrity but further avoid excessive tension build-up.

In an embodiment, the transversal waist barrier(s) (5) is elastic, preferably comprising an elastic material selected from the group consisting of an elastic film and one or more elastic strands. Advantageously this allows for application of tension that permits the barrier to stand upright in close contact to the skin when the article is worn and under stretch.

The elastic strands in any of the embodiments herein may be strand-coated with adhesive prior to joining to one or more layers such as the inner and outer nonwoven layers (9, 10) to form elastic panels (2, 3) and/or the backsheet (7), outer cover (7'), and/or outer nonwoven layer (10) to form the elastic waist barrier(s) (5). Advantageously this allows to join the one or more layers together by the said strand-coated elastic strands and by doing so limit the amount of adhesive used with resulting improved softness as well as reduced waste. Alternatively, or additionally, adhesive in the form of a plurality of stripes is applied such as by slot coating.

In a preferred embodiment, the one or more elastic strands of the transversal waist barrier(s) (5) comprise at least one flat elastic (such as elastics having a cross-section with an aspect ratio of longest to smallest dimension of greater than 1, preferably greater than 1.5, even more preferably greater than 2), preferably in combination with at least one or at least two round elastics (such as elastics having a cross-section with an aspect ratio of longest to smallest dimension of about 1). Although flat elastics are generally more expensive they are advantageous in providing better gasketing effects and comfort on the wearer's skin, by combining flat and normal/round elastics one can achieve the desired gasketing and comfort whist limiting the cost.

When one or more, preferably only one, flat elastic is used, it is preferably positioned adjacent to an apex of the transversal waist barrier(s) (5) that may be closest to a transversal edge of the insert (4) and further round elastic(s) positioned further from said apex compared to the flat elastic(s). Advantageously this allows for the flat elastic to be positioned at the closest contact position to the wearer with other elastics being inboard (or further away) therefrom hence contributing to the desired tensile strength yet permitting to lower the overall density/dtex of the flat elastic and hence also cost, with limited impact on performance.

When elastic strands are used they may have a dtex in the range of from 300 to 1500, preferably from 380 to 1150, preferably from 350 to 1100, preferably from 400 to 1000, even more preferably from 450 to 900. When elastic strands are used in all of the front and/or back panels (2, 3) and the waist barrier(s) (5), the elastic strands of said panel(s) (2, 3) have a dtex that is equal to or greater than, preferably greater than, that of the elastic strand(s) of said transversal waist barrier(s) (5). Preferably the elastic strand(s) of said transversal waist barrier(s) (5) have a dtex that is from 40% to 85%, preferably from 45% to 75%, even more preferably from 50% to 70%, of the dtex of the elastic strands of said panel(s) (2, 3). Advantageously, this allows the barrier(s) to stand up and provide a gasketing effect close to the skin of the wearer without flattening or adding excessive resistance to stretch that would otherwise impact fit and comfort. The tension differential that results entails that a lower tension in the barrier(s) compared to the belt(s) is generated to a degree that upon stretching of the belt(s) the barrier(s) stand-up away from said belt(s).

The tensile stress (N/m) of the entirety of the front and back panels (2, 3), respectively, may be profiled in order to provide the functional benefits of the present disclosure, such as ease of stretch and application, while also maintaining certain force during wear, to prevent the article from sagging after loading. When the elasticity of the front and back panels (2, 3) are provided by a plurality of elastic strands (11) running in the transverse direction, the tensile stress may be adjusted by one or more of the following methods; 1) elongation rate of the elastic strands (11); 2) density (dtex) of the elastic strands (11); 3) longitudinal pitch of multiple elastic strands (11); and 4) effective length of elasticity of the elastic strands (11) in the transverse direction. By elongation, "0% elongation" is meant the original length of the elastic member.

The front and back panels (2, 3) may each be divided into multiple zones spanning in the transverse direction and defined by its location from the distal edge to the proximal edge relative to the percentage of the seam length wherein the distal edge is considered 0% and the proximal edge is considered 100%. The multiple zones may be configured to provide different tensile stress, or different functions to the front and back panels (2, 3), respectively.

In an embodiment, one or more second elastic strands (12) are comprised directly or indirectly between the outer nonwoven layer (10) and the backsheet (7), preferably between a body-facing surface of the outer nonwoven layer (10) and a garment-facing surface of the backsheet (7). An example of an indirect elastic arrangement is the presence of an outer cover layer (7') as described herein (that would generally be interposed between the backsheet (7) and the one or more second elastic strands (12)), and an example of a direct elastic arrangement is the absence of an outer cover layer (7') as described herein. Advantageously this arrangement allows for a simple yet effective elastification of the barrier(s) permitting optimal gasketing effects.

Preferably, the one or more second elastic strands (12) extends along the entire transversal length of the transversal waist barrier(s) (5) and beyond, preferably crossing the imaginary longitudinal axis (y) in a direction substantially parallel to the imaginary transverse axis (x). Preferably the one or more second elastic strands (12) extends from a first position substantially adjacent a seam edge of the outer nonwoven layer (10) to a second position substantially adjacent an opposite seam edge of the outer nonwoven layer (10) with the imaginary longitudinal axis (y) being positioned therebetween, more preferably wherein the one or more second elastic strands (12) extends from said first position to said second position and crosses and/or overlaps the entire transversal waist barrier(s) (5) generally along the imaginary transverse axis (x). Advantageously this allows not only to form a standing barrier upon stretching but further aids better fit on the wearer's front and/or back waist.

In an embodiment, the absorbent core (8) comprises absorbent material selected from the group consisting of superabsorbent polymer particles and/or fibers; and cellulose fibers, preferably wherein said absorbent material is enclosed within a core wrap comprising top and bottom core wrap layers being a same nonwoven layer folded to sandwich the absorbent material therein or distinct nonwoven layers joined together to sandwich the absorbent material therein. The superabsorbent polymer particles and/or fibers may be comprised in an amount of greater than 50%wt, preferably greater than 60%wt, even more preferably greater than 70%wt, most preferably from 75% to 100%wt, by total weight of absorbent material.

In an embodiment, the back panel (3) is wider (generally in a direction substantially parallel to the longitudinal axis y) than the front panel (2) such that a length of the seams is substantially equal to a width (generally in a direction substantially parallel to the longitudinal axis y) of the front panel (2) and less than a width of the back panel (3); or wherein the front panel (2) is wider than the back panel (3) such that a length of the seams is substantially equal to a width of the back panel (3) and less than a width of the front panel (2). Advantageously this may permit comfort as well as improved protection compared to symmetric arrangements.

It is preferred that the backsheet (7) comprises a further outer cover layer (7') on a garment-facing surface thereof, preferably wherein said outer cover layer (7') comprising one or more nonwoven layers, and/or wherein the backsheet (7) comprises a further outer cover layer (7') that is shorter in the longitudinal direction (generally parallel to the longitudinal axis y) than the topsheet (6) and/or backsheet (7) such that at least a portion of an overlap length between the insert (4) and the panel (2, 3) is free of said outer cover layer (7') and preferably wherein the backsheet (7) is directly joined to, or is in contact with, the panel (2, 3) in said portion of an overlap length between the insert (4) and the panel (2, 3) free of said outer cover layer (7'). Advantageously this allows for improved softness in areas where needed and yet allow for material savings thus permitting both reduced cost and waste.

The outer cover layer (7') is preferably different from the inner and/or outer nonwoven layers (9, 10) meaning that they may have different properties selected from basis weight (in gsm) and composition. In an embodiment, the outer cover (7') comprises a spunbond nonwoven preferably comprising bicomponent fibers typically selected from the group consisting of polypropylene and polyethylene. The outer cover (7') preferably has a basis weight that is less than the basis weight of the inner and/or outer nonwoven layers (9, 10). The outer cover (7') may have a basis weight of from 8 g/m² (gsm) to 20 g/m² (gsm), preferably from 10 g/m² (gsm) to 15 g/m². Advantageously, the outer cover provides for softness to the touch to the care giver yet, as selected, it limits stress build-up particularly in embodiments with reduced length in the longitudinal direction as described above. Indeed without wishing to be bound by theory, stress build-up in the transition region corresponding to the terminal edge of the outer cover may result in a higher risk of tearing in the insert to panel joint.

In a preferred embodiment, the absorbent insert (4) is folded-over to form the waist barrier(s) (5) such that at least a portion of the topsheet (6) of the non-folded part of said insert (4) may come into contact with the folded part of said insert (4). Advantageously this allows the barrier to overlap the topsheet so as to help guide the exudates therethrough and into the core once stopped by said barrier.

Preferably, the waist barrier(s) (5) extends from 5% to 50%, preferably from 10% to 40%, even more preferably from 15% to 30%, of the total folded length (TFL) of the outer nonwoven layer (10) along the longitudinal axis (y). Advantageously, this may allow formation of a sufficiently large pocket yet avoid excessive resizing of the insert that may add cost and waste.

In an embodiment, the substantially liquid impermeable film layer or backsheet (7) comprises indicia, preferably a coloured print, at a position corresponding to the transversal waist barrier (5); and/or wherein the one or more second elastic strands (12) are coloured; and/or wherein a garment facing surface of the outer nonwoven layer (10) is coloured; and/or wherein the transversal waist barrier (5) comprises indicia selected from a coloured print and coloured adhesive (preferably comprising one or more pigments). Advantageously this allows the user or care giver to better visualise the barrier(s) so as to aid correct positioning and placement thereof onto a subject.

In an embodiment, the absorbent article further comprising a pair of longitudinally extending cuffs positioned along left and right longitudinal edges of the insert (4), and wherein the transversal waist barrier(s) (5) is positioned above said cuffs such that said barrier(s) (5) is closer to a wearer's skin than said cuffs; preferably wherein the cuffs are joined to a body-facing surface of the topsheet (6) and wherein each of said cuffs is folded and joined to itself on a body-facing surface thereof at a joining position (JP) corresponding to the transversal waist barrier(s) (5), preferably said joints or joining positions (JP) extending along a lengthwise portion of, and being substantially adjacent to, the left and/or right longitudinal edges of the insert (4). Advantageously this reduces risk of leakage multidirectionally and prevents leakage to seep through between the transversal barrier and cuffs unlike when otherwise arranged.

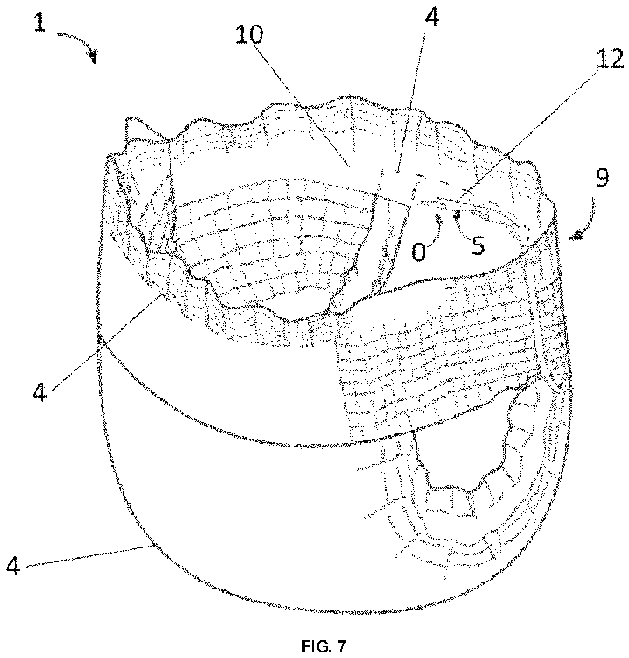

In an embodiment, for example shown in Fig.7, the barrier(s) (5) herein form an opening (0) (typically about a terminal edge of the folded outer nonwoven layer (10)) of an exudate containing pocket and wherein substantially the entire surface of the pocket that is congruent with a topsheet (6) of the insert (4) comprises a liquid impermeable layer preferably being a film, that may be an elastic film (generally according to the embodiments described herein).

Preferably, the thickness of the insert (4) in an overlap area of said waist barrier (5) (e.g. an area of the insert (4) overlapping with the waist barrier (5)) is less than the remaining thickness of said insert (4) (e.g. in areas other than the overlap area); and/or wherein the amount of absorbent material in in an overlap area of said waist barrier (5) is less (preferably at least 5%wt less, preferably at least 10%wt less, even more preferably at least 15%wt less, even more preferably at least 20%wt less) than the amount of absorbent material in other areas of said insert. Generally said overlap area further overlapping with the front and/or back panels respectively. Advantageously this allows for a larger pocket for exudate collection that synergistically cooperates with the overlapping front and/or back belts.

The insert (4) may comprise a topsheet selected from an embossed nonwoven and/or a carded air-through-bonded nonwoven. The embossed nonwoven may comprise a spunbond nonwoven. Advantageously the topographical surface of such particular nonwoven selection allows for the formation of peaks and slumps that acts to not only slow down the movement of liquid stool towards the transversal edges but further allows for containment ridges in areas overlapping the barrier(s) (5).

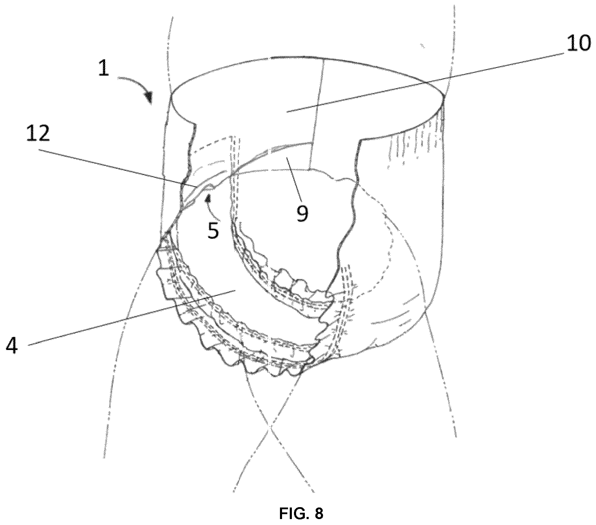

It is understood herein that all embodiments making reference to barrier(s) positioned at the back of the article, equally apply to the front of the article (i.e. all embodiments making reference to a barrier positioned with reference to a back transversal edge of the insert may be mirrored to provide similar such constructs at the front transversal edge). This is for example exemplified in Fig. 8. Advantageously, such pocket formation on the front may be effective in containing gushes of urine and/or to enclose therein male genitalia of a wearer. Front an back transversal barriers (5) as described herein may be combined to provide all-round exudate leakage protection.

### THE METHOD

Methods herein for the manufacture of an absorbent article (1) comprise the steps of: providing a front panel (2); providing a back panel (3); providing an absorbent insert (4) comprising front and back transversal edges and left and right longitudinal edges connecting the front and back transversal edges to form a perimeter thereof (and generally wherein the left and right longitudinal edges extend substantially along the longitudinal axis y and are oppositely disposed such that said longitudinal axis y extends therebetween, and the front and back transversal edges extend substantially along the transversal axis x, and typically substantially perpendicular to the longitudinal axis y, and are oppositely disposed such that said transverse axis x extends therebetween); folding a portion of said absorbent insert (4) proximal to the front and/or back transversal edges thereof onto itself, the fold being in the shape of a substantially U-fold, to form transversal waist barrier(s) (5); joining said insert (4) to said front and back panels (2, 3) (generally such that at least a portion of the insert (4) overlaps each of the front and back panels (2, 3) and forms a connection bridge between said front and back panels); and joining said front and back panels (2, 3) together along a pair of oppositely disposed side seams. Advantageously an absorbent article having transversal barrier(s) as described herein can be attained in a simple yet effective manner.

Generally the front and back panels herein are made from continuous webs that are subsequently severed into discrete front and back panels typically after the step of joining the insert (4) to said front and back panels (2, 3).

Preferably, the method further comprises the steps of folding an outer nonwoven layer (10) of the front and/or back panels (2, 3) over the folded absorbent insert (4) and/or an inner nonwoven layer (9) and joining said folded outer nonwoven layer (10) directly or indirectly to the folded absorbent insert (4). Advantageously this allows to prevent irritation or discomfort to the wearer as explained hereinabove whilst avoiding the use of added components/materials/elements whilst maintaining a simple and cost-effective production process.

### TEST METHODS

### Hysteresis Test Method

The Hysteresis Test can be used to various specified strain values. The Hysteresis Test utilizes a commercial tensile tester (e.g., from Instron Engineering Corp. (Canton, MA), SINTECH-MTS Systems Corporation (Eden Prairie, MN) or equivalent) interfaced with a computer. The computer isused to control the test speed and other test parameters and for collecting, calculating, and reporting the data. The tests are performed under laboratory conditions of 23 °C + 2°C and relative humidity of 50% + 2%. The specimens are conditioned for 24 hours prior to testing.

The specimen is cut with a dimension of 10 mm in the intended stretch direction of the ear X 25.4 mm in the direction perpendicular to the intended stretch direction of the ear. A specimen is collected from either an inelastic region or from an elastic region.
1. Select the appropriate grips and load cell. The grips should have flat surfaces and should be wide enough to grasp the specimen along its full width. Also, the grips should provide adequate force and suitable surface to ensure that the specimen does not slip during testing. The load cell is selected so that the tensile response from the specimen tested is between 25% and 75% of the capacity of the load cell used.
2. Calibrate the tester according to the manufacturer's instructions.
3. Set the distance between the grips (gauge length) at 7 mm.
4. Place the specimen in the flat surfaces of the grips such that the uniform width lies along a direction perpendicular to the gauge length direction. Secure the specimen in the upper grip, let the specimen hang slack, then close the lower grip. Set the slack preload at 5 gram/force. This means that the data collection starts when the slack is removed (at a constant crosshead speed of 13mm/min) with a force of 5 gram force. Strain is calculated based on the adjusted gauge length (lini), which is the length of the specimen in between the grips of the tensile tester at a force of 5 gram force. This adjusted gauge length is taken as the initial specimen length, and it corresponds to a strain of 0%. Percent strain at any point in the test is defined as the change in length relative to the adjusted gauge length, divided by the adjusted gauge length, multiplied by 100.
5(a) First cycle loading: Pull the specimen to the 100% strain at a constant cross head speed of 70 mm/min. Report the stretched specimen length between the grips as Imax.
5(b) First cycle unloading: Hold the specimen at the 100% strain for 30 seconds and then return the crosshead to its starting position (0% strain or initial sample length, lini) at a constant cross head speed of 70 mm/min. Hold the specimen in the unstrained state for 1 minute.
5(c) Second cycle loading: Pull the specimen to the 100% strain at a constant cross head speed of 70 mm/min.5(d) Second cycle unload: Next, hold the specimen at the 100% strain for 30 seconds and then return the crosshead to its starting position (i.e. 0% strain) at a constant cross head speed of 70 mm/min.

A computer data system records the force exerted on the sample during the test as a function of applied strain. From the resulting data generated, the following quantities are reported:
i. Length of specimen between the grips at a slack preload of 5 gram-force (Imi) to the nearest 0.001 mm.
ii. Length of specimen between the grips on first cycle at the 100% strain (Imax) to the nearest 0.001 mm.
iii. Length of specimen between the grips at a second cycle load force of 7 gram-force (lext) to the nearest 0.001 mm.
iv. % Set, which is defined as (lext - Imi) / (Imax - Imi) * 100% to the nearest 0.01%.

The testing is repeated for six separate samples and the average and standard deviation reported.

## Claims

1. An absorbent article (1) for personal hygiene, preferably a disposable pant, comprising:
a substantially transversely extending front panel (2);
a substantially transversely extending back panel (3); and
a substantially longitudinally extending absorbent insert (4) joined to each of said front and back panels (2, 3), said absorbent insert (4) comprising front and back transversal edges and left and right longitudinal edges connecting the front and back transversal edges to form a perimeter thereof;
wherein the front and back panels (2, 3) are joined together to define a pair of side seams, and **characterised in that** at least a portion of said absorbent insert (4) proximal to the front and/or back transversal edges thereof comprises a transversal waist barrier (5) comprising a substantially liquid impermeable film layer and wherein said insert (4) comprises one or more folds forming the transversal waist barrier (5), and wherein the impermeable film is breathable and has a basis weight of less than 18 g/m² and forms an integral part of the insert.

2. An absorbent article (1) according to Claim 1 wherein the substantially liquid impermeable film layer comprises, preferably consists of, a backsheet (7) of the absorbent insert (4) and preferably comprises a material comprising, or consisting of, a polyester, more preferably said material being selected from the group consisting of polyethylene, polylactic acid, and polypropylene.

3. An absorbent article (1) according to any of the preceding Claims wherein the at least portion of said absorbent insert (4) proximal to the front and/or back transversal edges thereof comprises one or more folds forming the transversal waist barrier(s) (5), wherein at least one of said folds is substantially U-shaped.

4. An absorbent article (1) according to any of the preceding Claims wherein the fold has a length (L) in a longitudinal direction running substantially parallel to a longitudinal axis (y), and wherein said insert (4) further comprises a pair of longitudinally extending cuffs positioned along left and right longitudinal edges thereof and joined thereto along a tack-down length (LT) extending from a position proximal to at least the back transversal edge to a tack-down terminal position (TP), and wherein the tack-down length (LT) is less than or equal to about 2L, preferably said cuffs being joined to a body-facing surface of the topsheet (6).

5. An absorbent article (1) according to Claim 4 wherein the tack-down length (LT) is from 0.5L to 2L, preferably from 0.6L to 1.9L, even more preferably from 0.7L to 1.5L, most preferably from 0.8L to 1.2L.

6. An absorbent article according to any of the preceding Claims wherein the insert (4) comprises a pair of longitudinally extending cuffs positioned along left and right longitudinal edges thereof, wherein each cuff comprises at least one elastic (cE), and wherein at least a terminal portion (TPe) of said elastic overlaps the transversal waist barrier (5).

7. An absorbent article according to any of the preceding Claims wherein the transversal waist barrier (5) comprises one or more second elastic strands (12) positioned at a distance (d) from a terminal edge (TE) of the transversal waist barrier (5) forming a pocket opening, and wherein the distance between said terminal edge (TE) and the elastic closest to said terminal edge is less than about 0.6L, preferably from 0.15L to 0.5L, even more preferably from 0.20 to 0.45L.

8. An absorbent article (1) according to any of the preceding Claims wherein the one or more second elastic strands (12) of the transversal waist barrier (5) have a first dtex and the front and back panels (2,3) comprise a plurality of elastics having a second dtex, wherein the first dtex is greater than the second dtex, and preferably wherein the second dtex is from 300dtex to 1200dtex.

9. An absorbent article (1) according to any of the preceding Claims wherein the transversal waist barrier (5) comprises one or more second elastic strands (12) and wherein at least one of said elastic strands is a flat elastic.

10. An absorbent article (1) according to any of the preceding Claims wherein the absorbent insert (4) comprises a substantially liquid permeable topsheet (6), a substantially liquid impermeable backsheet (7), and an absorbent core (8) sandwiched therebetween, preferably wherein the absorbent core (8) is positioned inboard of the perimeter of said absorbent insert (4) when viewed in a planar direction such that the one or more folds are substantially free of absorbent material.

11. An absorbent article (1) according to any of the preceding Claims wherein the font and/or back panels (2, 3) are elastic and comprise an inner nonwoven layer (9), an outer nonwoven layer (10), and an elastic material (11) sandwiched therebetween, wherein the elastic material is selected from the group consisting of an elastic film and a plurality of elastic strands.

12. An absorbent article (1) according to Claim 11 wherein the outer nonwoven layer (10) is wider than the inner nonwoven layer (9), generally in a direction substantially parallel to the longitudinal axis y, and is folded over said inner nonwoven layer (9) such that it overlaps the portion of said absorbent insert (4) proximal to the front and/or back transversal edges, preferably such that said outer nonwoven layer (10) overlaps both a portion of the backsheet (7) and a portion of the topsheet (6) that remains exposed from said one or more transversal waist barrier(s) (5) such to form a flange (F_{L}).

13. An absorbent article (1) according to any of the preceding Claims wherein the transversal waist barrier(s) (5) is elastic, preferably comprising an elastic material selected from the group consisting of an elastic film and one or more elastic strands.

14. An absorbent article (1) according to Claims 11 to 13 wherein one or more second elastic strands (12) are comprised directly or indirectly between the outer nonwoven layer (10) and the backsheet (7), preferably between a body-facing surface of the outer nonwoven layer (10) and a garment-facing surface of the backsheet (7).

15. An absorbent article (1) according to any of the preceding Claims wherein the absorbent core (8) comprises absorbent material selected from the group consisting of superabsorbent polymer particles and/or fibers; and cellulose fibers, preferably wherein said absorbent material is enclosed within a core wrap comprising top and bottom core wrap layers being a same nonwoven layer folded to sandwich the absorbent material therein or distinct nonwoven layers joined together to sandwich the absorbent material therein.

16. An absorbent article (1) according to any of the preceding Claims wherein the back panel (3) is wider than the front panel (2) such that a length of the seams is substantially equal to a width of the front panel (2) and less than a width of the back panel (3); or wherein the front panel (2) is wider than the back panel (3) such that a length of the seams is substantially equal to a width of the back panel (3) and less than a width of the front panel (2).

17. An absorbent article (1) according to Claims 2 to 16 wherein the backsheet (7) comprises a further outer cover layer (7') on a garment-facing surface thereof, preferably wherein said outer cover layer (7') comprising one or more nonwoven layers, and/or wherein the backsheet (7) comprises a further outer cover layer (7') that is shorter in the longitudinal direction than the topsheet (6) and/or backsheet (7) such that at least a portion of an overlap length between the insert (4) and the panel (2, 3) is free of said outer cover layer (7') and preferably wherein the backsheet (7) is directly joined to, or in contact with, the panel (2, 3) in said portion of an overlap length between the insert (4) and the panel (2, 3) free of said outer cover layer (7').

18. An absorbent article (1) according to any of the preceding Claims wherein the absorbent insert (4) is folded-over to form the waist barrier(s) (5) such that the topsheet (6) of the non-folded part of said insert (4) is able to come into contact with the folded part of said insert (4).

19. An absorbent article (1) according to Claims 12 to 18 wherein the waist barrier(s) (5) extends from 5% to 50%, preferably from 10% to 40%, even more preferably from 15% to 30%, of the total folded length (TFL) of the outer nonwoven layer (10) along the longitudinal axis (y).

20. An absorbent article (1) according to any of the preceding Claims wherein the substantially liquid impermeable film layer, or backsheet (7), comprises indicia, preferably a coloured print, at a position corresponding to the transversal waist barrier (5); and/or wherein the one or more second elastic strands (12) are coloured; and/or wherein a garment facing surface of the outer nonwoven layer (10) is coloured; and/or wherein the transversal waist barrier (5) comprises indicia selected from a coloured print and coloured adhesive.

21. An absorbent article (1) according to any of the preceding Claims further comprising a pair of longitudinally extending cuffs positioned along left and right longitudinal edges of the insert (4), and wherein the transversal waist barrier(s) (5) is positioned above said cuffs such that said barrier(s) (5) is closer to a wearer's skin than said cuffs; preferably wherein the cuffs are joined to a body-facing surface of the topsheet (6) and wherein each of said cuffs is folded and joined to itself on a body-facing surface thereof at a joining position (JP) substantially corresponding to the transversal waist barrier(s) (5) to form an exudate collection pocket.

22. A method for the manufacture of an absorbent article (1) comprising the steps of:
providing a front panel (2);
providing a back panel (3);
providing an absorbent insert (4) comprising front and back transversal edges and left and right longitudinal edges connecting the front and back transversal edges to form a perimeter thereof;
folding a portion of said absorbent insert (4) proximal to the front and/or back transversal edges thereof onto itself, in the shape of a substantially U-fold, to form transversal waist barrier(s) (5);
joining said insert (4) to said front and back panels (2, 3);
and joining said front and back panels (2, 3) together along a pair of oppositely disposed side seams.

23. A method according to Claim 22 further comprising the steps of folding an outer nonwoven layer (10) of the front and/or back panels (2, 3) over the folded absorbent insert (4) and joining said folded outer nonwoven layer (10) directly or indirectly to the folded absorbent insert (4).

## Patentansprüche

1. Absorbierender Artikel (1) für Körperhygiene, vorzugsweise eine Einwegunterhose, umfassend:
eine sich im Wesentlichen quer erstreckende Vorderseite (2);
eine sich im Wesentlichen quer erstreckende Rückseite (3); und
eine sich im Wesentlichen in Längsrichtung erstreckende absorbierende Einlage (4), die mit jeder der Vorder- und Rückseite (2, 3) verknüpft ist, die absorbierende Einlage (4) umfassend einen vorderen und einen hinteren Querrand und einen linken und einen rechten Längsrand, die den vorderen und den hinteren Querrand verbinden, um einen Umfang davon auszubilden;
wobei die Vorder- und Rückseite (2, 3) miteinander verknüpft sind, um ein Paar Seitennähte zu definieren, und **gekennzeichnet sind dadurch, dass** mindestens eines Abschnitt der absorbierenden Einlage (4) proximal zu dem vorderen und/oder dem hinteren Querrand eine Quertaillenbarriere (5) umfasst, umfassend eine im Wesentlichen flüssigkeitsundurchlässige Folienschicht, und wobei die Einlage (4) eine oder mehrere Falten umfasst, die die Quertaillenbarriere (5) ausbilden, und wobei die undurchlässige Folie atmungsaktiv ist und ein Flächengewicht von weniger als 18 g/m² aufweist und einen integralen Teil der Einlage ausbildet.

2. Absorbierender Artikel (1) nach Anspruch 1, wobei die im Wesentlichen flüssigkeitsundurchlässige Folienschicht eine Unterschicht (7) der absorbierenden Einlage (4) umfasst, vorzugsweise daraus besteht, und vorzugsweise ein Material umfasst, umfassend ein Polyester oder bestehend aus diesem, mehr bevorzugt das Material aus der Gruppe ausgewählt ist, bestehend aus Polyethylen, Polymilchsäure und Polypropylen.

3. Absorbierender Artikel (1) nach einem der vorstehenden Ansprüche, wobei der mindestens Abschnitt der absorbierenden Einlage (4) proximal zu dem vorderen und/oder dem hinteren Querrand davon eine oder mehrere Falten umfasst, die die Quertaillenbarriere(n) (5) ausbilden, wobei mindestens eine der Falten im Wesentlichen U-förmig ist.

4. Absorbierender Artikel (1) nach einem der vorstehenden Ansprüche, wobei die Falte eine Länge (L) in einer Längsrichtung aufweist, die im Wesentlichen parallel zu einer Längsachse (y) verläuft, und wobei die Einlage (4) ferner ein Paar sich in Längsrichtung erstreckenden Bündchen umfasst, die entlang des linken und des rechten Längsrands davon positioniert und daran entlang einer Festheftungslänge (LT) verknüpft sind, die sich von einer Position proximal zu mindestens dem hinteren Querrand bis zu einer Festheftungsendposition (TP) erstreckt, und wobei die Festheftungslänge (LT) kleiner als oder gleich etwa 2L ist, vorzugsweise wobei die Bündchen mit einer dem Körper zugewandten Oberfläche der Oberschicht (6) verknüpft sind.

5. Absorbierender Artikel (1) nach Anspruch 4, wobei die Festheftungslänge (LT) von 0,5 L bis 2 L, vorzugsweise von 0,6 L bis 1,9L, noch mehr bevorzugt von 0,7 L bis 1,5 L und am meisten bevorzugt von 0,8 L bis 1,2 L beträgt.

6. Absorbierender Artikel nach einem der vorstehenden Ansprüche, wobei die Einlage (4) ein Paar sich in Längsrichtung erstreckenden Bündchen umfasst, die entlang des linken und des rechten Längsrands davon positioniert sind, wobei jedes Bündchen mindestens ein Gummiband (cE) umfasst und wobei mindestens ein Endabschnitt (TPe) des Gummibands die Quertaillenbarriere (5) überlappt.

7. Absorbierender Artikel nach einem der vorstehenden Ansprüche, wobei die Quertaillenbarriere (5) einen oder mehrere zweite elastische Stränge (12) umfasst, die in einem Abstand (d) von einem Endrand (TE) der Quertaillenbarriere (5) positioniert sind und eine Taschenöffnung ausbilden, und wobei der Abstand zwischen dem Endrand (TE) und dem Gummiband, das dem Endrand am nächsten liegt, weniger als etwa 0,6 L, vorzugsweise von 0,15 L bis 0,5 L, noch mehr bevorzugt von 0,20 L bis 0,45 L beträgt.

8. Absorbierender Artikel (1) nach einem der vorstehenden Ansprüche, wobei der eine oder die mehreren zweiten elastischen Stränge (12) der Quertaillenbarriere (5) einen ersten dtex aufweisen und die Vorder- und Rückseite (2, 3) eine Vielzahl von Gummibändern umfassen, die einen zweiten dtex aufweist, wobei der erste dtex größer als der zweite dtex ist und vorzugsweise wobei der zweite dtex zwischen 300 dtex und 1200 dtex liegt.

9. Absorbierender Artikel (1) nach einem der vorstehenden Ansprüche, wobei die Quertaillenbarriere (5) einen oder mehrere zweite elastische Stränge (12) umfasst und wobei mindestens einer der elastischen Stränge ein flaches Gummiband ist.

10. Absorbierender Artikel (1) nach einem der vorstehenden Ansprüche, wobei die absorbierende Einlage (4) eine im Wesentlichen flüssigkeitsdurchlässige Oberschicht (6), eine im Wesentlichen flüssigkeitsundurchlässige Unterschicht (7) und einen absorbierenden Kern (8) umfasst, der dazwischengelegt ist, vorzugsweise wobei der absorbierende Kern (8) im Inneren des Umfangs der absorbierenden Einlage (4) positioniert ist, wenn in einer planaren Richtung derart betrachtet, dass die eine oder die mehreren Falten im Wesentlichen frei von absorbierendem Material sind.

11. Absorbierender Artikel (1) nach einem der vorstehenden Ansprüche, wobei die Vorder- und/oder Rückseite (2, 3) elastisch sind und eine innere Vliesschicht (9), eine äußere Vliesschicht (10) und ein elastisches Material (11) umfassen, das dazwischengelegt ist, wobei das elastische Material aus der Gruppe ausgewählt ist, bestehend aus einer elastischen Folie und einer Vielzahl von elastischen Strängen.

12. Absorbierender Artikel (1) nach Anspruch 11, wobei die äußere Vliesschicht (10) breiter als die innere Vliesschicht (9) ist, im Allgemeinen in einer Richtung, die im Wesentlichen parallel zu der Längsachse y verläuft, und über die innere Vliesschicht (9) derart gefaltet ist, dass sie den Abschnitt der absorbierenden Einlage (4) proximal zu dem vorderen und/oder dem hinteren Querrand überlappt, vorzugsweise derart, dass die äußere Vliesschicht (10) sowohl einen Abschnitt der Unterschicht (7) als auch einen Abschnitt der Oberschicht (6) überlappt, der von der einen oder den mehreren Querbarriere(n) (5) derart freiliegt, um einen Flansch (F_{L}) auszubilden.

13. Absorbierender Artikel (1) nach einem der vorstehenden Ansprüche, wobei die Quertaillenbarriere(n) (5) elastisch ist/sind, vorzugsweise umfassend ein elastisches Material, das aus der Gruppe ausgewählt ist, bestehend aus einer elastischen Folie und einem oder mehreren elastischen Strängen.

14. Absorbierender Artikel (1) nach den Ansprüchen 11 bis 13, wobei ein oder mehrere zweite elastische Stränge (12) direkt oder indirekt zwischen der äußeren Vliesschicht (10) und der Unterschicht (7) umfasst sind, vorzugsweise zwischen einer dem Körper zugewandten Oberfläche der äußeren Vliesschicht (10) und einer dem Kleidungsstück zugewandten Oberfläche der Unterschicht (7).

15. Absorbierender Artikel (1) nach einem der vorstehenden Ansprüche, wobei der absorbierende Kern (8) absorbierendes Material umfasst, das aus der Gruppe ausgewählt ist, bestehend aus superabsorbierenden Polymerpartikeln und/oder -fasern; und Zellulosefasern, vorzugsweise wobei das absorbierende Material von einer Kernhülle umschlossen ist, umfassend eine obere und eine untere Kernhüllenschicht, die eine gleiche Vliesschicht, die gefaltet ist, um das absorbierende Material darin zwischenzulegen, oder verschiedene Vliesschichten sind, die miteinander verknüpft sind, um das absorbierende Material darin zwischenzulegen.

16. Absorbierender Artikel (1) nach einem der vorstehenden Ansprüche, wobei die Rückseite (3) breiter als die Vorderseite (2) derart ist, dass eine Länge der Nähte im Wesentlichen gleich einer Breite der Vorderseite (2) und kleiner als eine Breite der Rückseite (3) ist; oder wobei die Vorderseite (2) breiter als die Rückseite (3) derart ist, dass eine Länge der Nähte im Wesentlichen gleich einer Breite der Rückseite (3) und kleiner als eine Breite der Vorderseite (2) ist.

17. Absorbierender Artikel (1) nach den Ansprüchen 2 bis 16, wobei die Unterschicht (7) eine weitere äußere Deckschicht (7') auf einer dem Kleidungsstück zugewandten Oberfläche umfasst, vorzugsweise wobei die äußere Deckschicht (7') eine oder mehrere Vliesschichten umfasst, und/oder wobei die Unterschicht (7) eine weitere äußere Deckschicht (7') umfasst, die in der Längsrichtung kürzer als die Oberschicht (6) und/oder die Unterschicht (7) derart ist, dass mindestens ein Abschnitt einer Überlappungslänge zwischen der Einlage (4) und der Seite (2, 3) frei von der äußeren Deckschicht (7') ist, und vorzugsweise wobei die Unterschicht (7) in dem Abschnitt einer Überlappungslänge zwischen der Einlage (4) und der Seite (2, 3), die frei von der äußeren Deckschicht (7') ist, mit der Seite (2, 3) direkt verknüpft ist oder mit dieser in Kontakt steht.

18. Absorbierender Artikel (1) nach einem der vorstehenden Ansprüche, wobei die absorbierende Einlage (4) umgefaltet ist, um die Taillenbarriere(n) (5) derart auszubilden, dass die Oberschicht (6) des nicht gefalteten Teils der Einlage (4) mit dem gefalteten Teil der Einlage (4) in Kontakt kommen kann.

19. Absorbierender Artikel (1) nach den Ansprüchen 12 bis 18, wobei sich die Taillenbarriere(n) (5) entlang der Längsachse (y) von 5 % bis 50 %, vorzugsweise von 10 % bis 40 %, noch mehr bevorzugt von 15 % bis 30 % der gesamten gefalteten Länge (TFL) der äußeren Vliesschicht (10) erstreckt.

20. Absorbierender Artikel (1) nach einem der vorstehenden Ansprüche, wobei die im Wesentlichen flüssigkeitsundurchlässige Folienschicht oder Unterschicht (7) an einer Position, die der Quertaillenbarriere (5) entspricht, Kennzeichnungen, vorzugsweise einen farbigen Aufdruck umfasst; und/oder wobei der eine oder die mehreren zweiten elastischen Stränge (12) gefärbt sind; und/oder wobei eine dem Kleidungsstück zugewandte Oberfläche der äußeren Vliesschicht (10) gefärbt ist; und/oder wobei die Quertaillenbarriere (5) Kennzeichen umfasst, die aus einem farbigen Aufdruck und einem farbigen Klebstoff ausgewählt sind.

21. Absorbierender Artikel (1) nach einem der vorstehenden Ansprüche, ferner umfassend ein Paar sich in Längsrichtung erstreckenden Bündchen, die entlang dem linken und dem rechten Längsrand der Einlage (4) positioniert sind, wobei die Quertaillenbarriere(n) (5) oberhalb der Bündchen derart positioniert ist/sind, dass die Barriere(n) (5) näher an einer Haut eines Trägers als die Bündchen liegt/liegen; vorzugsweise wobei die Bündchen an einer dem Körper zugewandten Seite des Oberschicht (6) verknüpft sind und wobei jedes der Bündchen an einer Verknüpfungsposition (JP), die im Wesentlichen der/den Quertaillenbarriere(n) (5) entspricht, gefaltet und mit sich selbst verknüpft ist, um eine Exsudatauffangtasche auszubilden.

22. Verfahren für die Herstellung eines absorbierenden Artikels (1), umfassend die Schritte:
Bereitstellen einer Vorderseite (2);
Bereitstellen einer Rückseite (3);
Bereitstellen einer absorbierenden Einlage (4), umfassend den vorderen und den hinteren Querrand und den linken und den rechten Längsrand, die den vorderen und den hinteren Querrand verbinden, um einen Umfang davon auszubilden;
Falten eines Abschnitts der absorbierenden Einlage (4) proximal zu dem vorderen und/oder dem hinteren Querrand davon auf sich selbst, in der Form einer im Wesentlichen U-förmigen Falte, um Quertaillenbarriere(n) (5) auszubilden;
Verknüpfen der Einlage (4) mit der Vorder- und Rückseite (2, 3);
und Verknüpfen der Vorder- und Rückseite (2, 3) miteinander entlang eines Paars von gegenüberliegend angeordneten Seitennähten.

23. Verfahren nach Anspruch 22, ferner umfassend die Schritte des Faltens einer äußeren Vliesschicht (10) der Vorder- und/oder Rückseite (2, 3) über die gefaltete absorbierende Einlage (4) und des direkten oder indirekten Verknüpfens der gefalteten äußeren Vliesschicht (10) mit der gefalteten absorbierenden Einlage (4).

## Revendications

1. Article absorbant (1) pour l'hygiène personnelle, de préférence une culotte jetable, comprenant :
un panneau avant (2) s'étendant sensiblement transversalement ;
un panneau arrière (3) s'étendant sensiblement transversalement ; et
un insert absorbant (4) s'étendant sensiblement longitudinalement, joint à chacun desdits panneaux avant et arrière (2, 3), ledit insert absorbant (4) comprenant des bords transversaux avant et arrière et des bords longitudinaux gauche et droit reliant les bords transversaux avant et arrière pour former un périmètre de celui-ci ;
dans lequel les panneaux avant et arrière (2, 3) sont joints ensemble pour définir une paire de coutures latérales, et **caractérisé en ce qu'**au moins une portion dudit insert absorbant (4) à proximité des bords transversaux avant et/ou arrière de celui-ci comprend une barrière à la taille transversale (5) comprenant une couche de film sensiblement imperméable aux liquides et dans lequel ledit insert (4) comprend un ou plusieurs plis formant la barrière à la taille transversale (5), et dans lequel le film imperméable est respirant et a un poids de base inférieur à 18 g/m² et forme une partie intégrante de l'insert.

2. Article absorbant (1) selon la revendication 1 dans lequel la couche de film sensiblement imperméable aux liquides comprend, de préférence consiste en, une feuille arrière (7) de l'insert absorbant (4) et comprend de préférence un matériau comprenant, ou constitué de, un polyester, plus préférablement ledit matériau étant choisi dans le groupe constitué de polyéthylène, d'acide polylactique, et de polypropylène.

3. Article absorbant (1) selon l'une quelconque des revendications précédentes dans lequel l'au moins une portion dudit insert absorbant (4) à proximité des bords transversaux avant et/ou arrière de celui-ci comprend un ou plusieurs plis formant la ou les barrières à la taille transversales (5), dans lequel au moins l'un desdits plis est sensiblement en forme de U.

4. Article absorbant (1) selon l'une quelconque des revendications précédentes dans lequel le pli a une longueur (L) dans une direction longitudinale se prolongeant sensiblement parallèlement à un axe longitudinal (y), et dans lequel ledit insert (4) comprend en outre une paire de manchettes s'étendant longitudinalement, positionnées le long de bords longitudinaux gauche et droit de celui-ci et jointes à celui-ci le long d'une longueur de rabattage (LT) s'étendant d'une position à proximité d'au moins le bord transversal arrière à une position terminale de rabattage (TP), et dans lequel la longueur de rabattage (LT) est inférieure ou égale à environ 2L, de préférence lesdites manchettes étant jointes à une surface faisant face vers le corps de la feuille supérieure (6).

5. Article absorbant (1) selon la revendication 4 dans lequel la longueur de rabattage (LT) est de 0,5 L à 2 L, de préférence de 0,6 L à 1,9 L, encore plus préférablement de 0,7 L à 1,5 L, le plus préférablement de 0,8 L à 1,2 L.

6. Article absorbant selon l'une quelconque des revendications précédentes dans lequel l'insert (4) comprend une paire de manchettes s'étendant longitudinalement positionnées le long de bords longitudinaux gauche et droit de celui-ci, dans lequel chaque manchette comprend au moins un élastique (cE), et dans lequel au moins une portion terminale (TPe) dudit élastique chevauche la barrière à la taille transversale (5).

7. Article absorbant selon l'une quelconque des revendications précédentes dans lequel la barrière à la taille transversale (5) comprend un ou plusieurs seconds brins élastiques (12) positionnés à une distance (d) d'un bord terminal (TE) de la barrière à la taille transversale (5) formant une ouverture de poche, et dans lequel la distance entre ledit bord terminal (TE) et l'élastique le plus proche dudit bord terminal est inférieure à environ 0,6 L, de préférence de 0,15 L à 0,5 L, encore plus préférablement de 0,20 L à 0,45 L.

8. Article absorbant (1) selon l'une quelconque des revendications précédentes dans lequel le ou les seconds brins élastiques (12) de la barrière à la taille transversale (5) ont un premier dtex et les panneaux avant et arrière (2, 3) comprennent une pluralité d'élastiques ayant un second dtex, dans lequel le premier dtex est supérieur au second dtex, et de préférence dans lequel le second dtex est de 300 dtex à 1 200 dtex.

9. Article absorbant (1) selon l'une quelconque des revendications précédentes dans lequel la barrière à la taille transversale (5) comprend un ou plusieurs seconds brins élastiques (12) et dans lequel au moins l'un desdits brins élastiques est un élastique plat.

10. Article absorbant (1) selon l'une quelconque des revendications précédentes dans lequel l'insert absorbant (4) comprend une feuille supérieure (6) sensiblement perméable aux liquides, une feuille arrière (7) sensiblement imperméable aux liquides, et un noyau absorbant (8) pris en sandwich entre celles-ci, de préférence dans lequel le noyau absorbant (8) est positionné à l'intérieur du périmètre dudit insert absorbant (4) lorsqu'il est vu dans une direction plane de telle sorte que le ou les plis sont sensiblement dépourvus de matériau absorbant.

11. Article absorbant (1) selon l'une quelconque des revendications précédentes dans lequel les panneaux avant et/ou arrière (2, 3) sont élastiques et comprennent une couche non tissée interne (9), une couche non tissée externe (10), et un matériau élastique (11) pris en sandwich entre celles-ci, dans lequel le matériau élastique est choisi dans le groupe constitué d'un film élastique et d'une pluralité de brins élastiques.

12. Article absorbant (1) selon la revendication 11 dans lequel la couche non tissée externe (10) est plus large que la couche non tissée interne (9), généralement dans une direction sensiblement parallèle à l'axe longitudinal y, et est repliée sur ladite couche non tissée interne (9) de telle sorte qu'elle chevauche la partie dudit insert absorbant (4) à proximité des bords transversaux avant et/ou arrière, de préférence de telle sorte que ladite couche non tissée externe (10) chevauche à la fois une portion de la feuille arrière (7) et une portion de la feuille supérieure (6) qui reste exposée à partir de ladite ou desdites barrières à la taille transversale(s) (5) de manière à former une collerette (F_{L}).

13. Article absorbant (1) selon l'une quelconque des revendications précédentes dans lequel la ou les barrières à la taille transversales (5) sont élastiques, de préférence comprenant un matériau élastique choisi dans le groupe constitué d'un film élastique et un ou plusieurs brins élastiques.

14. Article absorbant (1) selon les revendications 11 à 13 dans lequel un ou plusieurs seconds brins élastiques (12) sont compris directement ou indirectement entre la couche non tissée externe (10) et la feuille arrière (7), de préférence entre une surface faisant face au corps de la couche non tissée externe (10) et une surface faisant face au vêtement de la feuille arrière (7).

15. Article absorbant (1) selon l'une quelconque des revendications précédentes dans lequel le noyau absorbant (8) comprend un matériau absorbant choisi dans le groupe constitué de particules et/ou fibres de polymère superabsorbant ; et de fibres de cellulose, de préférence dans lequel ledit matériau absorbant est enfermé au sein d'une enveloppe de noyau comprenant des couches d'enveloppe de noyau supérieure et inférieure étant une même couche non tissée pliée pour prendre en sandwich le matériau absorbant à l'intérieur de celle-ci ou des couches non tissées distinctes jointes ensemble pour prendre en sandwich le matériau absorbant à l'intérieur de celles-ci.

16. Article absorbant (1) selon l'une quelconque des revendications précédentes dans lequel le panneau arrière (3) est plus large que le panneau avant (2) de telle sorte qu'une longueur des coutures est sensiblement égale à une largeur du panneau avant (2) et inférieure à une largeur du panneau arrière (3) ; ou dans lequel le panneau avant (2) est plus large que le panneau arrière (3) de telle sorte qu'une longueur des coutures est sensiblement égale à une largeur du panneau arrière (3) et inférieure à une largeur du panneau avant (2).

17. Article absorbant (1) selon les revendications 2 à 16 dans lequel la feuille arrière (7) comprend une autre couche de couverture externe (7') sur une surface faisant face au vêtement de celle-ci, de préférence dans lequel ladite couche de couverture externe (7') comprenant une ou plusieurs couches non tissées, et/ou dans lequel la feuille arrière (7) comprend une autre couche de couverture externe (7') qui est plus courte dans la direction longitudinale que la feuille supérieure (6) et/ou la feuille arrière (7) de telle sorte qu'au moins une partie d'une longueur de chevauchement entre l'insert (4) et le panneau (2, 3) est dépourvue de ladite couche de couverture externe (7') et de préférence dans lequel la feuille arrière (7) est directement jointe à, ou en contact avec, le panneau (2, 3) dans ladite partie d'une longueur de chevauchement entre l'insert (4) et le panneau (2, 3) dépourvue de ladite couche de couverture externe (7').

18. Article absorbant (1) selon l'une quelconque des revendications précédentes dans lequel l'insert absorbant (4) est replié pour former la ou les barrières à la taille (5) de telle sorte que la feuille supérieure (6) de la partie non pliée dudit insert (4) est apte à entrer en contact avec la partie pliée dudit insert (4).

19. Article absorbant (1) selon les revendications 12 à 18 dans lequel la ou les barrières à la taille (5) s'étendent de 5 % à 50 %, de préférence de 10 % à 40 %, encore plus préférablement de 15 % à 30 %, de la longueur totale pliée (TFL) de la couche non tissée externe (10) le long de l'axe longitudinal (y).

20. Article absorbant (1) selon l'une quelconque des revendications précédentes, dans lequel la couche de film sensiblement imperméable aux liquides, ou la feuille arrière (7), comprend des repères, de préférence une impression colorée, au niveau d'une position correspondant à la barrière à la taille transversale (5) ; et/ou dans lequel le ou les seconds brins élastiques (12) sont colorés ; et/ou dans lequel une surface faisant face au vêtement de la couche non tissée externe (10) est colorée ; et/ou dans lequel la barrière à la taille transversale (5) comprend des repères choisis parmi une impression colorée et un adhésif coloré.

21. Article absorbant (1) selon l'une des revendications précédentes comprenant en outre une paire de manchettes s'étendant longitudinalement positionnées le long de bords longitudinaux gauche et droit de l'insert (4), et dans lequel la ou les barrières à la taille transversales (5) sont positionnées au-dessus desdites manchettes de telle sorte que ladite ou lesdites barrières (5) sont plus proches de la peau d'un porteur que lesdites manchettes ; de préférence dans lequel les manchettes sont jointes à une surface faisant face au corps de la feuille supérieure (6) et dans lequel chacune desdites manchettes est pliée et jointe à elle-même sur une surface faisant face au corps de celle-ci au niveau d'une position de jonction (JP) correspondant sensiblement à la ou aux barrières à la taille transversales (5) pour former une poche de collecte des exsudats.

22. Procédé pour la fabrication d'un article absorbant (1) comprenant les étapes consistant à :
fournir un panneau avant (2) ;
fournir un panneau arrière (3) ;
fournir un insert absorbant (4) comprenant des bords transversaux avant et arrière et des bords longitudinaux gauche et droit reliant les bords transversaux avant et arrière pour former un périmètre de celui-ci ;
plier une partie dudit insert absorbant (4) à proximité des bords transversaux avant et/ou arrière de celui-ci sur elle-même, sous la forme d'un pli sensiblement en U, pour former une ou des barrières à la taille transversales (5) ;
joindre ledit insert (4) auxdits panneaux avant et arrière (2, 3) ;
et joindre lesdits panneaux avant et arrière (2, 3) ensemble le long d'une paire de coutures latérales disposées de manière opposée.

23. Procédé selon la revendication 22 comprenant en outre les étapes consistant à plier une couche non tissée externe (10) des panneaux avant et/ou arrière (2, 3) sur l'insert absorbant (4) plié et à joindre ladite couche non tissée externe (10) pliée directement ou indirectement à l'insert absorbant (4) plié.
